# EUROPÄISCHE PATENTSCHRIFT

(11) **EP 0 546 209 B1**
(45) Veröffentlichungstag und Bekanntmachung des Hinweises auf die Patenterteilung: **02.11.1995**
(21) Anmeldenummer: 91121221.5
(22) Anmeldetag: 11.12.1991
(51) Int. Cl.: A61B 17/39

(54) **Endoskopische Zange**
Endoscopic forceps
Pince endoscopique

(43) Veröffentlichungstag der Anmeldung: 16.06.1993
(73) Patentinhaber: Storz, Karl, Dr.med. h.c., D-78532 Tuttlingen (DE)
(72) Erfinder:
(74) Vertreter: Witte, Alexander, Dr.-Ing.

(56) Entgegenhaltungen:
- EP-A- 0 066 465
- DE-A- 2 734 847
- FR-A- 2 310 137
- FR-A- 2 526 303

## Beschreibung

Die Erfindung betrifft eine endoskopische Zange zur Anwendung von Hochfrequenzströmen.

Es sind bereits endoskopische Zangen oder auch andere Vorrichtungen mit einem Generator und einer Elektrode zum Hochfrequenz-Schneiden und/oder Koagulieren oder zur Laser-Applikation bekannt, bei denen der Anschluß des Hochfrequenzkabels durch einen Steckerstift oder eine Steckerbuchse am Griffstück der Zange vorgesehen ist. Dadurch steht aber das Griffstück unter der Einwirkung des Hochfrequenzstromes, was sowohl vom Anwender als auch von den Kontrollbehörden beanstandet wird.

Ferner ist bei einem bipolaren Operationsinstrument mit einem schleifenförmigen Operationselement am patientennahen Ende zum Schneiden, insbesondere in der Blase, mittels eines hochfrequenten Stromes bekannt, bei dem die zweite Elektrode in das Instrument geführt und gegenüber der ersten Elektrode isoliert ist. (Schweizer Patent 566 790, DE - GM 87 07 820).

Aus der nächstliegenden Druckschrift des Standes der Technik FR-A-2 310 137 ist eine bipolare Koagulationszange zur Anwendung von Hochfrequenzströmen bekannt, bei der die beiden Leiter in einem rohrförmigen Element geführt sind, die am patientennahen Ende mit zwei Mauleilen in Verbindung stehen, die aufgrund einer radialen Vorspannung die Tendenz haben, sich radial aufzuspreizen. Am patientenfernen Ende enden die beiden Leiter in einem bipolaren Stecker zum Anschluß an eine Stromquelle. Über das rohrförmige Element ist ein weiteres rohrförmiges Element geschoben, das mit einem der beiden Griffstücke der Zange verbunden ist. Das die Leiter tragende innere rohrförmige Element ist über eine Halterung fest mit dem anderen der beiden Griffstücke der Zange verbunden. Die beiden Griffstücke sind über ein Achsgelenk miteinander verbunden. Das äußere rohrförmige Element kann beim Betätigen der Griffstücke derart über das innere rohrförmige Element verschoben werden, daß es in Richtung der radial gespreizten Maulteile bewegt wird, sich dabei über die radial gespreizten Maulteile schiebt und diese, radial nach innen gerichtet, schließt. Das über das innere rohrförmige Element geschobene äußere rohrförmige Element arbeitet somit als Betätigungselement für die Maulteile.

Aus der DE-A-2 734 847 ist eine Koagulationszange mit wenigstens zwei von einem Kontaktträger am proximalen Ende vorstehenden, gegeneinander elektrisch isolierenden Kontaktzungen bekannt, wobei ein den Kontaktträger mit den Zungen axial verschiebbar aufnehmendes Metallrohr vorgesehen ist, welches beim Verschieben in Richtung auf das proximale Ende der Kontaktzungen diese zangenartig schließt. Ein Griff nimmt die elektrischen Anschlüsse auf und trägt Betätigungselemente für die Relativverschiebung von Metallrohr und Kontaktzungen. Der Griff besteht aus einem Bügel, dessen eines Ende relativ unverschieblich mit dem Kontaktträger in Verbindung steht, das andere Ende dagegen mit dem verschieblichen Metallrohr in Verbindung steht. Beim Drücken der Bügelenden aufeinander zugerichtet, wird das Metallrohr derart verschoben, daß die radial gespreizten Kontaktzungen radial zusammengedrückt und somit geschlossen werden.

Aus der FR-A-2 526 303 ist eine Koagulationszange bekannt, bei der die Maulteile der Zange über einen Betätigungsstab bewegbar sind. Der Betätigungsstab ist in einem rohrförmigen Schaft eines Griffstückes eingeschoben, von dessen Außenseite, etwa rechtwinklig zur Schaftachse ein Stecker für eine Steckverbindung mit einem Hochfrequenzkabel absteht. Der Schaft mündet endseitig einstückig mit dem Grifstück. Ein weiteres relativ dazu bewegliches Griffstück ist über ein Achsgelenk mit dem anderen Griffstück verbunden.

Aus der EP-A-0 066 465 ist eine Zange zur Durchführung von Biopsien bekannt. Eine Stange, die am patientennahen Ende mit radial gespreizten Maulteilen versehen ist, ist am patientenfernen Ende mit einem Griffstück der Zange verbunden. Dieses Griffstück ist über eine Achsgelenkverbindung mit einem zweiten Griffstück verbunden, das mit einem über die die Maulteile tragende Stange verschiebbaren Rohr derart in Verbindung steht, daß beim Betätigen der Griffelemente das Rohr längs der Stange verschoben werden kann. Wird das Rohr in Richtung der radial gespreizten Maulteile verschoben, so werden diese vom Rohr radial nach innen gerichtet zusammen gedrückt und geschlossen. Aus diesem Dokument ist das Problem bekannt, daß, falls die Maulteile zum festen Ergreifen stark gegeneinander gedrückt werden sollen, die entsprechend über die Griffstücke ausgeübten Kräfte dazu führen können, daß sich Stange samt überschiebbarem Rohr relativ zu den Griffstücken wippen können.

Aufgabe der Erfindung ist hier Abhilfe zu schaffen und eine endoskopische Zange zu schaffen, bei der die Griffstücke einerseits nicht unter der Einwirkung des Hochfrequenzstromes stehen können, so daß der Arzt damit ungefährdet arbeiten kann, und die derart ausgestaltet ist, daß sie den Anforderungen des täglichen Gebrauchs gewachsen ist.

Erfindungsgemäß wird die Aufgabe durch eine endoskopische Zange zur Anwendung von Hochfrequenzströmen, mit einem rohrförmigen Schaft gelöst, in dem ein Zangenmaulteile tragender Betätigungsstab bewegbar angeordnet ist, der mit zwei Griffstücken am patientenfernen Ende in Verbindung steht, wobei der Betätigungsstab am patientenfernen Ende als Stecker für eine Steckverbindung mit einem Hochfrequenzkabel ausgebildet ist, und der Betätigungsstab gegenüber dem Schaft und den Griffstücken isoliert ist, wobei Schenkel der beiden Griffstücke über zwei Achsgelenke miteinander verbunden sind, die als Rundbolzen ausgebildet sind, welche in entsprechende Ausnehmungen der Griffteile eingreifen.

Vorteilhafte Ausbildungen sind durch die Unteransprüche gekennzeichnet.

Dadurch ist unter anderem die Montage und Demontage der erfindungsgemäßen Zange sehr leicht.

Weitere Vorteile und Einzelheiten der Erfindung ergeben sich aus der nun folgenden Beschreibung unter Hinweis auf die Zeichnung, In dieser zeigen:
- Fig. 1: Eine Seitenansicht auf das patientenferne Ende einer Zange,
- Fig. 2: eine Seitenansicht auf einen stark vergrößerten Ausschnitt im Bereich der Gelenkverbindung von Griffstücken der Zange, und
- Fig. 3: eine teilweise geschnittene Draufsicht auf die Ausführungsform nach der Fig. 2.

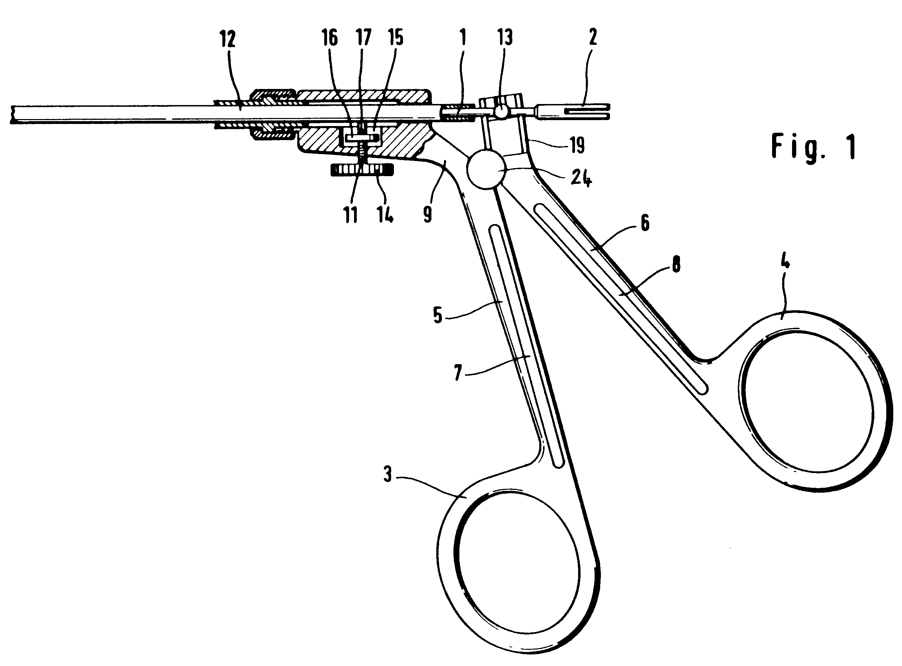

Bei Fig. 1 handelt es sich um eine endoskopische Zange, deren nicht dargestellte Maulteile am patientennahen linken Ende sitzen und dem Fachmann bestens bekannt sind. Es ist dem Fachmann auch schon bekannt, daß die Maulteile durch einen Betätigungsstab 1 betätigt werden, der gegenüber den feststehenden Schaft 12 durch das bewegliche Griffstück 4 nach links und rechts hin- und herbewegt wird.

Da dies im Prinzip alles bekannt ist, wird es nicht im einzelnen dargestellt.

Der Zangenschaft 12 ist erfinderungsgemäß ebenso wie die Griffstücke 3 und 4 gegenüber dem Betätigungsstab 1 isoliert, weil dieser an seinem patientenfernen Ende mit dem Hochfrequenzstecker 2 versehen ist, der in bekannter Weise durch den aus Metall bestehenden Betätigungsstab 1 den Hochfrequenzstrom zu dem hier nicht dargestellten patientennahen Ende nach links leitet.

Diese Isolierung kann zum Beispiel dadurch geschehen, daß der Schaft 12 vollständig aus einem isolierenden Kunststoff besteht, während auch die beiden Griffstücke 3 und 4 aus Kunststoff bestehen können.

Hierzu ist in der Fig. 1 dargestellt, daß der Schaft 12 durch eine Rändelschraube 14 in dem oberen Schenkel 9 des feststehenden Griffstückes 3 festgeklemmt ist. Der Bolzen 11 der Rändelschraube 14 durchdringt nämlich den Schenkel 9 und drückt auf den Schaft 12. Damit die Rändelschraube nicht verlorengeht, ist in einer Ausnehmung 15 eine Scheibe 16 angeordnet, die durch einen Stift in dem Gewindeteil 11 der Rändelschraube 14 gesichert ist.

Weiter rechts sieht man den Verbindungsbolzen 13, durch den der Betätigungsstab 1 mit dem oberen Schenkel 19 des Griffstückes 4 verbunden ist.

Darunter sieht man, daß in die beiden aus Kunststoff bestehenden Griffstücke 3 und 4 Metallteile 7 und 8 eingegossen oder eingespritzt sind, die zur Verstärkung dieser Teile und der Zange insgesamt dienen. Bekanntlich ist der Kunststoff ein wenig elastisch und den gelegentlich hohen Beanspruchungen einer solchen Zange sonst nicht gewachsen.

Aus dem gleichen Grund kann der Schraubkopf der Rändelschraube 14 ebenfalls aus Kunststoff bestehen, während der Bolzen 11 sowie die Scheibe 16 aus Metall gefertigt sein können.

Um die Zange nach der Erfindung zu demontieren, ist lediglich nötig, die Rändelschraube 14 sowie den Verbindungsbolzen 13 zu lösen. Danach läßt sich der Kunststoffschaft 12 zusammen mit dem aus Metall bestehenden Betätigungsstab 1 leicht entfernen, auch kann man danach den Betätigungsstab 1 leicht aus dem Schaft 12 herausziehen.

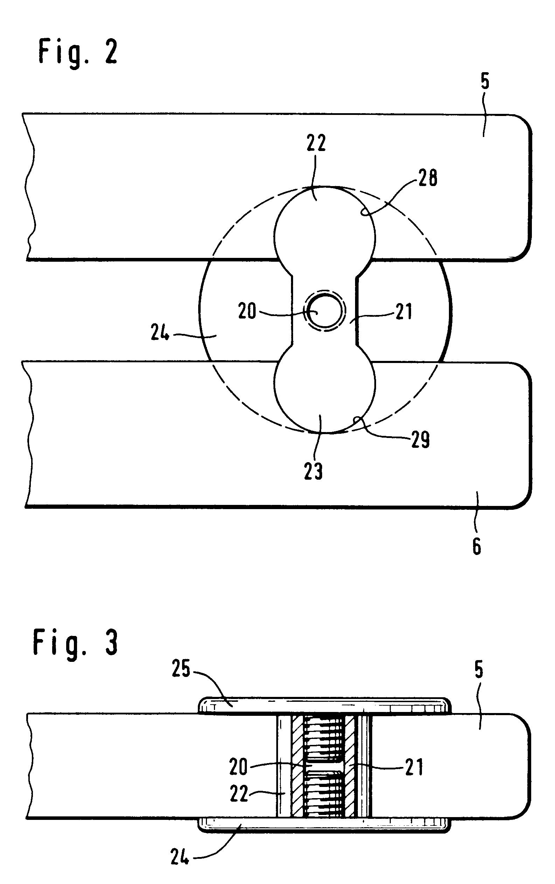

Fig. 2 zeigt eine Seitenansicht auf die Gelenkanordnung der Gelenkverbindung zwischen den Griffstücken. Fig. 2 zeigt nur diese Gelenkverbindung in stark vergrößertem Maßstab.Die Gelenkteile aus Kunststoff erfordern bei diesen medizinischen Zangen gegenüber den bekannten Ausführungen aus Metall eine besondere Stabilisierung, um den Anforderungen des täglichen Gebrauches gewachsen zu sein.

Gemäß der Fig.2 ist hierzu die Gelenkachse gemäß der Erfindung zweiachsig gestaltet. Die beiden Achsen bestehen gemäß der Fig. 2 aus zwei Bolzen 22 und 23, die in entsprechende Ausnehmungen der Schenkel 5 und 6 eingreifen. Zwischen diesen beiden Bolzen befindet sich ein die beiden Bolzen verbindendes Mittelstück 21, um das Mittelstück 21 mit den Bolzen zusammen zu halten.

Es besteht auch die Möglichkeit, dieses Mittelstück 21 mit den Rundbolzen , die hier nur zur Hälfte verwendet sind, als eine Einheit aus einen vollen Metallstück auszubilden. Der Fig. 2 kann man darüberhinaus entnehmen, daß in dem Mittelstück 21 eine Durchgangsbohrung 20 vorgesehen ist, um die Abdeckschrauben 24 und 25 nach der Fig. 3 einbringen zu können.

Der Fig. 3 kann man ferner entnehmen, daß die beiden Abdeckschrauben 24 und 25 mit einem verhältnismäßig großen Kopf oder einer Scheibe versehen sind, damit die Anordnung des Mittelstückes 21 vollständig abgedeckt wird. (Siche auch Fig 1).

Diese Ausführungsform zeigt den Vorteil, daß die Handkraft durch die beiden Rundbolzen 22 und 23 besser als bisher auf die beiden aus Kunststoff bestehenden Schenkel 5 und 6 übertragen werden kann.

Die Erfindung ist nicht auf die dargestellten Ausführungsformen beschränkt. Der Fachmann hat vielmehr die Möglichkeit, Abweichungen hiervon im Rahmen der Ansprüche zu erstellen.

## Patentansprüche

1. Endoskopische Zange zur Anwendung von Hochfrequenzströmen, mit einem rohrförmigen Schaft (12), in dem ein Zangenmaulteile tragender Betätigungsstab (1) bewegbar angeordnet ist, der mit zwei Griffstücken (3, 4) am patientenfernen Ende in Verbindung steht, wobei der Betätigungsstab (1) am patientenfernen Ende als Stecker (2) für eine Steckverbindung mit einem Hochfrequenzkabel ausgebildet ist und der Betätigungsstab (1) gegenüber dem Schaft (12) und den Griffstücken (3, 4) isoliert ist, wobei Schenkel (5, 6) der beiden Griffstücke (3, 4) über zwei Achsgelenke miteinander verbunden sind, die als Rundbolzen (22, 23) ausgebildet sind, welche in entsprechende Ausnehmungen (28, 29) der Griffstücke (3, 4) eingreifen.

2. Endoskopische Zange nach Anspruch 1, dadurch gekennzeichnet, daß die Rundbolzen (22, 23) mit einem Mittelstück (21) eine Einheit bilden.

3. Endoskopische Zange nach Anspruch 2, dadurch gekennzeichnet, daß das Mittelstück (21) eine Durchgangsbohrung (20) zur Aufnahme von Abdeckschrauben (24, 25) aufweist.

4. Endoskopische Zange nach einem der Ansprüche 1 bis 3, dadurch gekennzeichnet, daß die Griffstücke (3, 4) als Kunststoffteile ausgebildet sind.

5. Endoskopische Zange nach Anspruch 4, dadurch gekennzeichnet, daß die Schenkel (5, 6) der Griffstücke (3, 4) mit Metallteilen (7, 8) verstärkt sind.

6. Endoskopische Zange nach einem der Ansprüche 1 bis 5, dadurch gekennzeichnet, daß in einem starren Schenkel (9) des Griffstückes (3) eine Rändelschraube (14) zur Befestigung des Schaftes (12) angeordnet ist.

7. Endoskopische Zange nach Anspruch 6, dadurch gekennzeichnet, daß in dem Schenkel (9) eine Ausnehmung (15) zur Aufnahme einer Scheibe (16) angeordnet ist, die durch einen Stift (17) in einer Bohrung der Rändelschraube (14) gesichert ist.

8. Endoskopische Zange nach einem der Ansprüche 1 bis 7, dadurch gekennzeichnet, daß der Schaft (12) der Zange aus isolierendem Kunststoff ausgebildet ist.

## Claims

1. Endoscopic forceps for applying high-frequency currents, having a tubular shaft (12) in which there is movably disposed an actuating rod (1) which carries jaw parts of the forceps and is connected to two handle pieces (3, 4) at the end remote from the patient, wherein the actuating rod (1) is constructed, at the end remote from the patient, as a plug (2) for plug-in type connection to a high-frequency cable and the actuating rod (1) is insulated in relation to the shaft (12) and the handle pieces (3, 4), and wherein shanks (5, 6) on the two handle pieces (3, 4) are connected to one another via two spindle joints which are constructed as round bolts (22, 23) that engage in corresponding recesses (28, 29) in the handle pieces (3, 4).

2. Endoscopic forceps according to claim 1, characterized in that the round bolts (22, 23) form one unit with a central piece (21).

3. Endoscopic forceps according to claim 2, characterized in that the central piece (21) has a continuous bore (20) for receiving covering screws (24, 25).

4. Endoscopic forceps according to one of claims 1 to 3, characterized in that the handle pieces (3, 4) are constructed as plastics parts.

5. Endoscopic forceps according to claim 4, characterized in that the shanks (5, 6) of the handle pieces (3, 4) are reinforced with metal parts (7, 8).

6. Endoscopic forceps according to one of claims 1 to 5, characterized in that a knurled-head screw (14) for fixing the shaft (12) is disposed in a rigid shank (9) on the handle piece (3).

7. Endoscopic forceps according to claim 6, characterized in that a recess (15) for receiving a washer (16) which is secured by a pin (17) in a bore in the knurled-head screw (14) is disposed in the shank (9).

8. Endoscopic forceps according to one of claims 1 to 7, characterized in that the shaft (12) of the forceps is constructed from insulating plastics material.

## Revendications

1. Pince endoscopique pour l'application de courants à haute fréquence, comportant une tige (12) tubulaire dans laquelle est placée mobile une barre d'actionnement (1) portant des mâchoires de pince, laquelle barre est en liaison, à l'extrémité éloignée du patient, avec deux poignées (3, 4), la barre d'actionnement (1) étant configurée, à l'extrémité éloignée du patient, en connecteur (2) pour une connexion avec un câble à haute fréquence et la barre d'actionnement (1) étant isolée par rapport à la tige (12) et aux poignées (3, 4), des branches (5, 6) des deux poignées (3, 4) étant reliées entre elles par deux articulations à axe, qui sont réalisées sous la forme de chevilles rondes (22, 23), qui s'engagent dans des évidements (28, 29) correspondants des poignées (3, 4).

2. Pince endoscopique selon la revendication 1, caractérisée en ce que les chevilles rondes (22, 23) forment une unité avec une pièce centrale (21).

3. Pince endoscopique selon la revendication 2, caractérisée en ce que la pièce centrale (21) présente un trou débouchant (20) destiné à loger des vis de couverture (24, 25).

4. Pince endoscopique selon l'une des revendications 1 à 3, caractérisée en ce que les poignées (3, 4) sont des pièces en matière synthétique.

5. Pince endoscopique selon la revendication 4, caractérisée en ce que les branches (5, 6) des poignées (3, 4) sont renforcées avec des pièces métalliques (7, 8).

6. Pince endoscopique selon l'une des revendications 1 à 5, caractérisée en ce que dans une branche (9) fixe de la poignée (3) est placée une vis moletée (14) destinée à la fixation de la tige (12).

7. Pince endoscopique selon la revendication 6, caractérisée en ce que dans la branche (9) est prévu un évidement (15) destiné à loger une rondelle (16), qui est bloquée par une goupille (17) dans un perçage de la vis moletée (14).

8. Pince endoscopique selon l'une des revendications 1 à 7, caractérisée en ce que la tige (12) de la pince est en matière synthétique isolante.
